(19) Europäisches Patentamt
European Patent Office
Office européen des brevets

(11) Publication number: 0 532 759 A1

(12) **EUROPEAN PATENT APPLICATION published in accordance with Art. 158(3) EPC**

(21) Application number: **92905743.8**

(51) Int. Cl.[5]: **C07C 43/23**

(22) Date of filing: **05.03.92**

(86) International application number: **PCT/JP92/00262**

(87) International publication number: **WO 92/15543 (17.09.92 92/24)**

(30) Priority: **05.03.91 JP 62523/91**

(43) Date of publication of application: **24.03.93 Bulletin 93/12**

(84) Designated Contracting States: **AT BE CH DE DK ES FR GB GR IT LI LU MC NL SE**

(71) Applicant: **TSUMURA & CO.**
**4-10, Nihonbashi 3-chome**
**Chuo-ku, Tokyo 103(JP)**

(72) Inventor: **TAKEDA, Shuichi, 3586, Yoshiwara**
**Ami-machi**
**Inashiki-gun**
**Ibaraki 300-11(JP)**
Inventor: **ABURADA, Masaki, Tsumura & Co.**
**12-7, Nibancho**
**Chiyoda-ku**
**Tokyo 102(JP)**
Inventor: **ASAMI, Akitoshi, Tsumura & Co.**
**3586, Yoshiwara**
**Ami-machi Inashiki-gun**
**Ibaraki 300-11(JP)**
Inventor: **ISHIHARA, Kazuhisa, Tsumura & Co.**
**3586, Yoshiwara**
**Ami-machi Inashiki-gun**
**Ibaraki 300-11(JP)**
Inventor: **FUJIWARA, Tadami, Tsumura & Co.**
**12-7, Nibancho**
**Chiyoda-ku**
**Tokyo 102(JP)**
Inventor: **ICHIKAWA, Yukinobu, Tsumura & Co. 12-7, Nibancho**
**Chiyoda-ku**
**Tokyo 102(JP)**

(74) Representative: **Stuart, Ian Alexander et al**
**MEWBURN ELLIS 2 Cursitor Street**
**London EC4A 1BO (GB)**

(54) **NOVEL ALLYL ALCOHOL DERIVATIVE.**

(57) A novel allyl alcohol derivative represented by formula (I), which is useful for treating hepatic diseases, psoriasis, cancer, arteriosclerosis, thrombosis and ache.

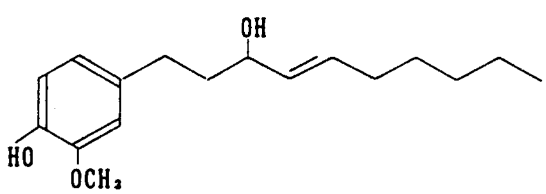

I

EP 0 532 759 A1

TECHNICAL FIELD

The present invention relates to a novel allyl alcohol derivative having a hepatopathy ameliorating activity, an activity for inhibiting the worsening of pathology related to keratosis and vasculavization and an inflammatory disease innibitory activity.

BACKGROUND ART

Several allyl alcohol derivatives having a pharmacological activity have hitherto been isolated from a hepatic metabolite.

Further, it is known that (6)-shogaol is useful as a therapeutic agent for keratosis (for example, see Japanese Unexamined Patent Publication (Kokai) No. 1-207256).

DISCLOSURE OF THE INVENTION

The present inventors have made extensive and intensive studies with a view to synthesizing a novel allyl alcohol derivative having a useful pharmacological activity. As a result, they have succeeded in synthesizing a novel substance having a hepathopathy ameliorating activity, a fibroblast growth inhibitory activity, a vascular endothelial cell growth inhibitory activity, an activity for inhibiting inflammation derived from a 5-lipoxygenase inhibitory activity, a platelet aggregation inhibitory activity and an analgesic activity, which has led to the completion of the present invention.

The present invention provides a novel allyl alcohol derivative represented by the following formula I (hereinafter referred to as "compound of the present invention"):

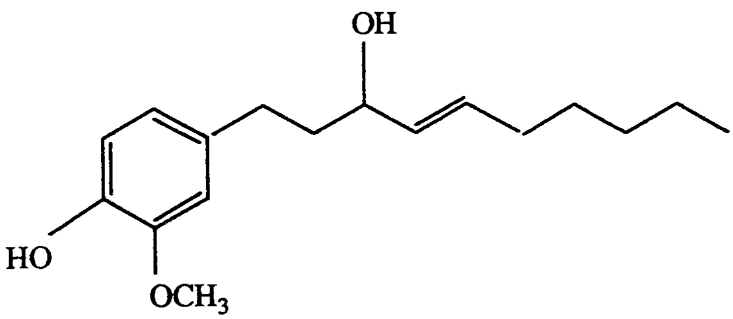

I

BEST MODE FOR CARRYING OUT THE INVENTION

The compound of the present invention is prepared through the use of (6)-shogaol as a starting compound, and (6)-shogaol can be prepared by the following process.

2 kg of a dried rhizome of zingiber officinale roscoe is extracted with an organic solvent such as an ether, ethanol or methanol to give a extract which is then subjected to fractionation with a mixed solution comprising organic solvents such as chloroform, ethyl acetate, benzene, acetone, n-hexane, ether and acetone and purified by various chromatography techniques.

Specific examples of the process for producing (6)-shogaol will now be described.

[Specific Example]

2 kg of a dried rhizome of zingiber officinale roscoe was pulverized and extracted twice under reflux with 3 liters of an ether for 7 hr. The extraction residue was further extracted twice under reflux with 3 liters of an ether for 7 hr. The extracts thus obtained were combined with each other, and the ether was removed under reduced pressure to give 77.05 g of an extract.

77.05 g of the extract obtained with the ether was applied to column chromatography wherein use was made of 700 g of silica gel (Kieselgel 60 manufactured by Merck) and development was conducted through the use of a mixed solvent comprising n-hexane and an ether while gradually increasing the proportion of the ether.

A fraction eluted with 0.6 liter of n-hexane-ether (70 : 30) was combined with a fraction eluted with 1.6 liters of n-hexane-ether (60 : 40), and the solvent was removed to give 13.6 g of a residue. The residue was applied again to column chromatography wherein use was made of silica gel to obtain a fraction eluted with n-hexane-ether (95 : 5), and the solvent was removed to give 5.48 g of a residue. The residue was applied to thin layer chromatography [plate: Kieselgel 60 PF254, development solvent: n-hexane-acetone (7 : 3)] to give 0.803 g (yield: 0.04 %) of a pale yellow oily substance. The physicochemical properties of the pale yellow oleaginous substance were in agreement with those of (6)-shogaol described in a literature.

A process for synthesizing the compound of the present invention will now be described.

Specifically, the compound of the present invention can be synthesized by a process properly selected from a process which comprises reacting (6)-shogaol prepared above in the presence of a catalyst such as palladium chloride, platinum, palladium, palladium hydroxide, rhodium or bis(triphenylphosphine)palladium chloride through the use of a reducing agent such as hydrogen, cyclohexadiene, ammonium formate or hydrazine in a general solvent such as ethyl acetate, benzene, toluene, methanol, tetrahydrofuran or dioxane at a temperature of 0 to 100 °C for 5 hr or more, a process which comprises reacting (6)-shogaol with sodium borohydride, sodium cyanoborohydride or tetra-n-butyl ammonium hydride in an alcohol such as methanol, ethanol or isopropanol at a temperature of 0 °C or above for 10 min or more, and a process which comprises reacting (6)-shogaol with an aluminum hydride such as aluminum lithium hydride, diisobutylaluminum hydride, bis(methoxyethoxy)aluminum sodium hydride in a solvent comprising an ether compound such as an ether, tetrahydrofuran or dimethoxyethane or a hydrocarbon such as benzene or toluene at a temperature around -80 °C to room temperature for 10 min or more.

The compound of the present invention thus prepared has a hepatopathy ameliorating activity, a keratinocyte cell growth inhibitory activity, a vascular endothelial cell growth inhibitory activity, an activity for inhibiting inflammation derived from a 5-lipoxygenase inhibitory activity and a cyclooxygenase inhibitory activity, a platelet aggregation inhibitory activity and an analgesic activity, which renders the compound of the present invention useful as a therapeutic drug for a hepatic disease, psoriasis, cancer, arterial sclerosis, thrombosis and pain.

One example of the process for producing the compound of the present invention will now be described in detail.

[Production Example]

34 mg of (6)-shogaol was dissolved in 10 ml of methanol, sodium borohydride (one microspatulas) was added thereto with ice cooling, and the mixture was stirred by means of a stirrer for 15 min. After the completion of the reaction, 5 drops of 2 N HCl were added, the solvent was removed by distillation, and the residue was purified by silica gel column chromatography (solvent: chloroform/ethyl acetate = 20/1) to give 32 mg of the compound of the present invention.

The compound of the present invention produced by the above-described process had the following physicochemical properties.

Infrared absorption spectrum $\nu^{CHC13}$ ($cm^{-1}$):
3552 (-OH), 2928, 2860 (C-H)
Mass spectrum (m/z): 278 ($M^+$), 137
Proton nuclear magnetic resonance spectrum:
(δppm in $CDCl_3$):
0.89 (3H, t, 1-$CH_3$),
1.2-1.5 (6H, m, $CH_2$ x 3),
1.7-2.1 (2H, 5-$CH_2$),
2.5-2.7 (4H, m, 9, 10-$CH_2$),
3.86 (3H, s, 17-$CH_3$),
4.07 (1H, ddd, J = 6.6, 6.6, 7Hz, 8-H),
5.49 (1H, ddt, J = 4.7, 15Hz, 7-H),
5.55 (1H, br, s, phenolic OH),
5.65 (1H, dddd, J = 1, 7, 7, 15Hz, 6-H),
6.68 (1H, dd, J = 2, 8Hz, 16-H),
6.70 (1H, d, J = 2Hz, 12-H),
6.82 (1H, d, J = 7Hz, 15-H)

The function of the compound of the present invention will now be described with reference to the following Experimental Examples.

[Experimental Example 1] 5-Lipoxygenase inhibitory activity

RBL-1 culture cells were suspended in a 50 mM phosphate buffer (pH: 7.4) containing 1 mM ethylenediaminetetraacetic acid (EDTA) and 10 % ethylene glycol so that the concentration was 5 x $10^6$ cells/ml. The suspension was subjected to an ultrasonic treatment and centrifuged at 10,000 x G for 10 min and further at 105,000 x G for 60 min. The resultant supernatant was used as a 5-lipoxygenase enzyme preparation.

50 mM phosphate buffer, 15 mM calcium chloride, 20 µM indomethacin, 18 mM glutathione, 6 mM adenosine triphosphate (ATP), 10 µM arachidonic acid, the enzyme preparation prepared above and an ethanol solution of the compound of the present invention were placed in a test tube in such a manner that the whole quantity was 0.6 ml and the final concentration was 5 µM, 1 µM and 0.5 µM. A reaction was allowed to proceed at 37° C for 10 min. After the completion of the reaction, 1.2 ml of acetone and 100 µl of 2 N formic acid were added thereto to terminate the reaction. 10 µl of 0.25 M n-butyl-3,5-dinitrobenzoate was added thereto as an internal standard, and extraction was conducted with 1.8 ml of n-hexane. The resultant extract was concentrated and dissolved in methanol, and the content of 5-hydroperoxyeicosatetraenoic acid (HETE) in the solution was measured by means of high performance liquid chromatography [column = TSK-gel ODS-80 TM (manufactured by Tosoh Corporation); mobile phase = acetonitrile : water : acetic acid (60 : 40 : 0.02); flow rate = 1 ml/min; ultraviolet rays for detection = 235 nm]. This content was regarded as an enzymatic activity. With respect to these results, the inhibition at each concentration was calculated by the following equation to determine 50 % inhibition concentration (hereinafter referred to as "$IC_{50}$").

$$\text{Inhibition (\%)} \ \frac{C - S}{C} \times 100$$

wherein C represents the peak area of 5-HETE in the case where no compound of the present invention has been contained (corrected by internal standard) ; and $ represents the peak area of 5-HETE in the case where the compound of the present invention has been added (corrected by internal standard).

As a result, the $IC_{50}$ value of the compound according to the present invention was 0.54 µM.

[Experimental Example 2] Cyclooxygenase inhibitory activity

60 ml of a 0.1 M phosphate buffer (pH: 7.5) containing 1 mM glutathione was added to 7.0 g of medulla of kidney of a rabbit, and the mixture was homogenized through the use of a polytron. The resultant homogenate was centrifuged at 9,000 x G for 20 min to obtain a microsome fraction. This fraction was dissolved in 7 ml of a 0.1 M phosphate buffer (pH: 7.5) containing 1 µM glutathione to give a cyclooxygenase enzyme preparation.

4 mM glutathione, 10 mM tryptophan, 0.25 µM hemoglobin (each in final concentration) and a phosphate buffer (pH: 7.5) were added to 15 µl of the enzyme preparation thus prepared, and [1-$^{14}$C] arachidonic acid (5 x $10^4$ dpm) and the compound of the present invention were further added thereto in such a manner that the concentrations were 40 µM, 20 µM, 10 µM and 5 µM, respectively (total amount: 200 µM). The mixture was incubated at 37° C for 15 min, and 1 N hydrochloric acid was added thereto to terminate the reaction. Prostaglandin $E_2$ ($PGE_2$) was added thereto as a carrier, and the reaction product was extracted with 2 ml of an ether. The resultant extract was concentrated and applied to thin layer chromatography (development solvent = chloroform : methanol : acetic acid = 18 : 1 : 1) to separate a product. Color development was conducted through the use of iodo vapor, and a portion corresponding to $PGE_2$ was scratched off. The $PGE_2$ radioactivity was measured by means of a liquid scintillation counter. With respect to these results, the inhibition at each concentration was calculated by the following equation to determine $IC_{50}$.

$$\text{Inhibition (\%)} \ \frac{C - S}{C} \times 100$$

wherein C represents the amount of formation of $PGE_2$ in the case where no compound of the present invention has been contained (corrected by internal standard) ; and S represents the amount of formation of $PGE_2$ in the case where the compound of the present invention has been added (corrected by internal standard).

As a result, the $IC_{50}$ value of the compound according to the present invention was 7.62 μM.

[Experimental Example 3] Activity for inhibiting growth of epidermal cell growth factor (EGF)

A culture solution (hereinafter referred to as "CS DMEM") containing 10 % calf serum adjusted in such a manner that fibroblast (balb/c 3T3 cells) was contained in a concentration of 2.5 x $10^4$ cells per 0.5 ml was incubated on a 24-hole plate in an amount of 1 ml per hole. After the fibroblast was saturated in a monolayer state, cultivation was conducted at 37°C for 12 hr in 1% CS DMEM instead of the above medium through the use of 5 % $CO_2$. 0.5 μg per hole of the compound of the present invention and 50 pg per hole of mouse EGF were added thereto, and the cultivation was further conducted for 16 hr. $^3$H-thymidine in an amount of 0.5 μCi/50 μl/well was added thereto, and the mixture was cultivated for 8 hr. Then, the culture solution was removed, and washing was conducted with a cooled isotonic phosphate buffer. A 5 % cold trichloroacetic acid was added thereto, and the mixture was allowed to stand for 30 min. The supernatant was removed, and the residue was dissolved in a 0.5 M sodium hydroxide solution. The solution was neutralized with 0.5 M hydrochloric acid, a scintillation cocktail was added thereto, and counting was conducted by means of a liquid scintillation counter. The radioactivity of $^3$H-thymidine incorporated into the cells accompanying the growth was measured to study the function of the novel allyl alcohol on the growth of the cell. As a result, a 72.9 % growth inhibition was observed on balb/c 3T3 cells of which the growth had been accelerated by EGF. From the above-described results, it was confirmed that the novel allyl alcohol had an excellent therapeutic activity for keratosis.

[Experimental Example 4] Action on proliferation and migration of human cell microvascular endothelial cell

5 x $10^4$ microvascular endothelial cells derived from human caul were placed in an upper chamber of a Boyden chamber, and 10 ng/ml of EGF was added as a control to a lower chamber separated from the upper chamber by means of a filter having a pore size of 5 μm (a polycarbonate filter manufactured by Nucleobore). 10 ng/ml of EGF and 10 μM/ml of the compound of the present invention were added to the drug administration group . The number of cells which had come out into the lower chamber was measured in terms of cells per HPF (hour per four field). The group to which the compound of the present invention has been added exhibited an percentage inhibition of 82 %, which rendered the compound of the present invention useful as a therapeutic agent for cancer and psoriasis associated with angiogenesis as an angiogenic inhibitor.

[Experimental Example 5] Arachidonic acid ear lobe edema inhibiting test

1 mg of the novel allyl alcohol derivative per 20 μl of acetone were applied to the auricle on the inside of the right of an ICR mouse (female, 8 - 11 weeks old). 30 min after the application, 10 μl of an acetone solution containing 2 mg of arachidonic acid was applied to the same site, and one hour after the application of the acetone solution, the thickness of the ear was measured by means of a dial thickness gauge. An equal weight of acetone was applied to the auricle on the left side, and measurement was conducted in the same manner as that described above, and (thickness on the left side) - (thickness on the right side) was regarded as swelling of the ear. Further, a group wherein the solvent was applied instead of the sample was used as a control, and the ear lobe edema inhibition was determined by the following equation.

$$\text{Inhibition (\%)} = \frac{\text{control group} - \text{sample administration group}}{\text{control group}} \times 100$$

The novel allyl alcohol derivative application group exhibited an 83 % edema inhibition as compared with the control, which rendered the novel allyl alcohol derivative useful as an anti-inflammatory drug.

[Experimental Example 6] Platelet aggregation inhibition test

Blood was collected from a rat subjected to fasting for 12 hr so that the volume of 3.8 % sodium citrate became 1/10. The collected blood was centrifuged at room temperature and 159 x G for 10 min to collect a platelet-rich plasma (PRP). The remaining blood was centrifuged at room temperature and 1800 x G for 10 min to collect PPP (platelet poor plasma). The number of platelets was measured by means of PC-603A manufactured by Erma and adjusted to 3 x $10^8$/ml with PPP. The aggregation capability was measured according to the Born method with the concentration of PRP being 0 % (PPP) and 100 % (PPP) through the use of a 4 channel NKK HEMATRACER aggregation meter. In an in vitro test, the novel allyl alcohol derivative was added in a concentration of 5 µM or 20 µM, the mixture was pre-incubated at 37°C for 3 min. An aggregation inducer was added thereto, and the aggregation capability was measured. Adenine diphosphate (ADP), collagen, epinephrine and arachidonic acid were used as the aggregation inducer. The concentration of the aggregation inducer was 2 µM for ADP, 1 µg/ml for collagen, 400 µM for arachidonic acid and 6 µg/ml of epinephrine. The novel ally] alcohol derivative was applied to the experiment after dissolution in dimethylsulfoxide (DMSO). The final concentration of DMSO thereof was 0.4 %, the addition of DMSO alone in this concentration had no clear effect on the aggregation of the platelet.

The results are given in the following Table 1.

Table 1

| Aggregation inducer | Platelet aggregation inhibition (%) | |
|---|---|---|
| | Concentration of novel allyl alcohol (µM) | |
| | 5 | 20 |
| ADP | 12 | 26 |
| collagen | 21 | 43 |
| epinephrine | 52 | 63 |
| arachidonic acid | 67 | 96 |

Since the novel allyl alcohol inhibited the reaction derived from the four platelet aggregation inducers, it was confirmed that it was useful as a therapeutic agent for thrombosis.

[Experimental Example 7] Analgesic activity test

Experimental was made with "Randall-Selitto" analgesic effect measuring apparatus (manufactured by Ugo Basile in Italy), pressure was applied through a pressure terminal, and the pressure (g) applied at the time showing a wriggling motion in rat was measured as a threshold value for pain. The threshold value for pain was measured for each leg, and rats showing substantially the same degree of threshold value for pain were selected and used for the experiment. A 20 % aqueous Brewer's yeast solution was subcutaneously administered in an amount of 0.1 ml to the right hind paw of the rat, and the threshold value for pain was measured on both legs 30 min and 60 min after the administration of the aqueous solution. At the same time, a suspension of the compound of the present invention in a 2 % polysorbate 80 physiological saline (prepared by dissolving the compound of the present invention in polysorbate 80 in an amount of 2 % of the whole amount of the solution and gradually adding and mixing physiological saline therewith) was intravenously and orally administered, and the threshold value for pain of both legs was measured 15 min, 30 min, 60 min, 90 min and 120 min after the administration. The measurements were compared with the control group, and an increase in the threshold value for pain was regarded as an evidence for the analgesic activity. After the administration of the compound of the present invention at a dose of 5 mg/kg, a significant increase ($p < 0.05$) in the threshold value for pain was observed within 15 to 60 min as compared with the control group.

Therefore, the relief of the pain by the compound of the present invention rendered the compound of the present invention useful as an analgesic agent.

The results of measurement of the analgesic activity according to the Randall-Selitto method are as given in Table 2.

Table 2

| | Threshold value for pain (g) | | |
|---|---|---|---|
| | 15 min | 30 min | 60 min |
| Control (n = 12) | 123 | 115 | 108 |
| Compound of the present invention, 70 mg/kg (n = 10) | $153^{a}$ | $183^{b}$ | $142^{a}$ |
| Compound of the present invention, 140 mg/kg (n = 10) | $190^{b}$ | $222^{b}$ | $210^{b}$ |
| Aminopyrine, 140 mg/kg (n = 9) | $190^{b}$ | $235^{b}$ | $212^{b}$ |
| Note) a : $p < 0.05$ b : $p < 0.01$ | | | |

[Experimental Example 8] $LD_{50}$ of the compound of the present invention

A suspension of the compound of the present invention in a 2 % polysorbate 80 physiological saline (prepared by dissolving the compound of the present invention in polysorbate 80 in an amount of 2 % of the whole amount of the solution and gradually adding and mixing physiological saline therewith) was intravenously and orally administered to a mouse, and 72 hr after the administration, the $LD_{50}$ was calculated based on whether the mouse had survived or was dead. In the calculation, the up-and-down method was used for the intravenous administration, while the Litchfield-Wilcoxon method was used for the oral administration.

The results were as given in Table 3.

Table 3

| Administration method | Number of animals | $LD_{50}$ mg/kg |
|---|---|---|
| Intravenous | 10 | 75.6 |
| Oral | 40 | 1342.4 |

The dose and formulation of the compound of the present invention will now be described.

The compound of the present invention can be administered in itself or together with a general preparation carrier to animals. The dosage form is not particularly limited and can be properly selected according to need. Examples of the dosage form include oral preparations such as tablets, capsules, granules, fine subtilaes and powders and parenteral preparations such as injections and suppositories.

In order to exhibit the intended effect as an oral preparation, it would be generally suitable that the compound of the present invention is dividedly administered several times at a dose of 10 to 100 mg per day per adult although the dose varies depending upon the age, weight and severity of disease of the patient.

Oral preparations such as tablets, capsules and granules are produced according to a conventional method through the use of, for example, starch, lactose, saccharose, mannite, carboxymethyl cellulose, corn starch or an inorganic salt. In these kinds of preparations, binders, disintegrators, surfactants, lubricants, fluidity accelerators, corrigents, colorants, perfumes, etc., may be properly used besides the above-described excipients. Specific examples of these additives will now be described.

[Binder]

Starch, dextrin, powdered acacia, gelatin, hydroxypropyl starch, methyl cellulose, carboxymethyl cellulose sodium, hydroxypropyl cellulose, crystalline cellulose, ethyl cellulose, polyvinyl pyrrolidone and macrogol.

[Disintegrator]

Starch, hydroxypropylstarch, sodium carboxymethyl cellulose, calcium carboxymethyl cellulose, carboxymethyl cellulose and lowly substituted hydroxypropyl cellulose.

[Surfactant]

Sodium laurylsulfate, soybean lecithin, sucrose fatty acid ester and polysorbate 80.

[Lubricant]

Talc, wax, hydrogenated vegetable oil, sucrose fatty acid ester, magnesium stearate, calcium stearate, aluminum stearate and polyethylene glycol.

[Fluidity accelerator]

Precipitated silicic acid anhydride, dried aluminum hydroxide gel, synthetic aluminum silicate and magnesium silicate.

Further, the compound of the present invention can be administered also in the form of a suspension, an emulsion, a syrup or an elixir. These various dosage forms may contain corrigents, stabilizers and colorants.

In order to attain the intended effect when the compound of the present invention is administered in the form of a parenteral preparation, it would be generally suitable that the compound of the present invention is administered through intravenous injection, intravenous drip, subcutaneous injection or intramuscular injection at a dose of 0.1 to 10 mg per day per adult although the dose varies depending upon the age, weight and severity of disease of the patient.

The parenteral agent can be produced according to a conventional method. In this case, distilled water for injection, physiological saline, aqueous glucose solution, vegetable oil for injection, sesame oil, peanut oil, soybean oil, corn oil, propylene glycol, polyethylene glycol, etc., may be generally used as a diluent. Further, if necessary, germicides, preservatives and stabilizers may be added. From the viewpoint of stability, the parenteral preparation may be used as follows. Specifically, the parenteral preparation is filled into a vial and frozen, water is removed by a conventional lyophilization technique, and a solution is prepared again from the lyophilized preparation immediately before use. Further, if necessary, it is also possible to properly add tonicity agents, stabilizers, preservatives, soothing agents, etc. Examples of other parenteral preparation include liquids for external use, liniments such as ointments and suppositories for intrarectal administration. These as well may be produced by a conventional method.

Examples of preparations wherein use is made of the compound of the present invention will now be described.

[Preparation Example 1]

| | |
|---|---|
| (1) Corn starch | 23.5 g |
| (2) Crystalline cellulose | 15 g |
| (3) Calcium carboxymethyl cellulose | 5 g |
| (4) Precipitated silicic acid anhydride | 0.5 g |
| (5) Magnesium stearate | 1 g |
| (6) Compound of the present invention | 5 g |
| Total | 50 g |

According to the above-described formulation, the components (1) to (6) were homogeneously mixed with each other, and the mixture was compression-molded by means of a tableting machine into tablets having a weight of 200 mg per tablet.

The tablet contained the compound of the present invention in an amount of 20 mg per tablet. It is dividedly administered several times at a dose of 1 to 4 tablets per day per adult.

[Preparation Example 2]

| | |
|---|---|
| (1) Crystalline cellulose | 39.5 g |
| (2) Magnesium stearate | 0.5 g |
| (3) Calcium carboxymethyl cellulose | 5 g |
| (4) Compound of the present invention | 5 g |
| Total | 50 g |

According to the above-described formulation, the components (1) and (4) and part of the component (2) were homogeneously mixed with each other, and the mixture was subjected to compression molding and pulverized. The component (3) and the remaining amount of the component (2) were added and mixed therewith. The mixture was compression-molded by means of a tableting machine into tablets having a weight of 200 mg per tablet.

The tablet contained the compound of the present invention in an amount of 20 mg per tablet. It is dividedly administered several times at a dose of 1 to 4 tablets per day per adult.

[Preparation Example 3]

| | |
|---|---|
| (1) Crystalline cellulose | 17 g |
| (2) 10 % Hydroxypropyl cellulose ethanol solution | 25 g |
| (3) Calcium carboxymethyl cellulose | 2.7 g |
| (4) Magnesium stearate | 0.3 g |
| (5) Compound of the present invention | 5 g |
| Total | 50 g |

According to the above-described formulation, the components (1), (2) and (5) were homogeneously mixed with each other, and the mixture was kneaded by a conventional method and granulated by means of an extruding granulator. The granule was dried and crushed, and the components (3) and (4) were mixed therewith. The mixture was compression-molded by means of a tableting machine into tablets having a weight of 200 mg per tablet.

The tablet contained the compound of the present invention in an amount of 20 mg per tablet. It is dividedly administered several times at a dose of 1 to 4 tablets per day per adult.

[Preparation Example 4]

| | |
|---|---|
| (1) Corn starch | 43 g |
| (2) Magnesium stearate | 0.5 g |
| (3) Calcium carboxymethyl cellulose | 1 g |
| (4) Precipitated silicic acid anhydride | 0.5 g |
| (5) Compound of the present invention | 5 g |
| Total | 50 g |

According to the above-described formulation, the components (1) to (5) were homogeneously mixed with each other, and the mixture was subjected to compression molding by means of a compression molding machine, pulverized by means of a pulverizer and sieved to give granules.

1 g of the granule contained 20 mg of the compound of the present invention. It is dividedly administered several times at a dose of 1 to 4 g per day per adult.

[Preparation Example 5]

| | |
|---|---|
| (1) Crystalline cellulose<br>(2) 10 % Hydroxypropyl cellulose ethanol solution<br>(3) Compound of the present invention | 29 g<br>20 g<br>1 g |
| Total | 50 g |

According to the above-described formulation, the components (1) to (3) were homogeneously mixed with each other, and the mixture was subjected to kneading and granulated by means of an extruding granulator. The granules were dried and sieved to give a granule preparation.

1 g of the granules contained 20 mg of the compound of the present invention. It is dividedly administered several times at a dose of 1 to 4 g per day per adult.

[Preparation Example 6]

| | |
|---|---|
| (1) Corn starch<br>(2) Precipitated silicic acid anhydride<br>(5) Compound of the present invention | 44.5 g<br>0.5 g<br>5 g |
| Total | 50 g |

According to the above-described formulation, the components (1) to (3) were homogeneously mixed with each other, and 200 mg of the mixture was filled into a No. 2 capsule.

1 g of the capsule contained 20 mg of the compound of the present invention. It is dividedly administered several times at a dose of 1 to 4 capsules per day per adult.

[Preparation Example 7]

| | |
|---|---|
| (1) Distilled water for injection<br>(2) Glucose<br>(3) Compound of the present invention | suitable amount<br>200 mg<br>1 mg |
| Total amount | 5 ml |

The components (2) and (3) were dissolved in distilled water for injection, and the solution was poured into an ampoule having a capacity of 5 ml and sterilized at 121°C for 15 min under pressure to give an injection.

[Preparation Example 8]

| | |
|---|---|
| (1) Compound of the present invention | 0.05 g |
| (2) White petrolatum | 25 g |
| (3) Stearyl alcohol | 22 g |
| (4) Bleached beeswax | 15 g |
| (5) Polyoxyethylene (25) monostearic ester | 2.3 g |
| (6) Sorbitan monopalmitate | 2.7 g |
| (7) Methyl p-oxybenzoate | 0.05 g |
| (8) Propyl p-oxybenzoate | 0.05 g |
| (9) Purified water | 32.85 g |
| Total | 100 g |

According to the above-described formulation, the components (1) to (9) were homogeneously mixed with each other, and the mixture was heat-melted to give an ointment.

## Claims

**1.** A novel ally] alcohol derivative represented by the following formula I:

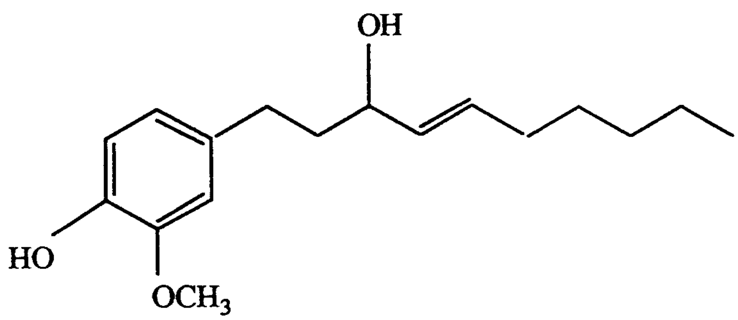

I

**2.** A pharmaceutical composition comprising as an effective component a compound according to claim 1.

**3.** A method of treating a hepatic disease, comprising administering an effective amount of a compound according to claim 1.

**4.** A method of treating psoriasis, comprising administering an effective amount of a compound according to claim 1.

**5.** A method of treating cancer, comprising administering an effective amount of a compound according to claim 1.

**6.** A method of treating arterial sclerosis, comprising administering an effective amount of a compound according to claim 1.

**7.** A method of treating thrombosis, comprising administering an effective amount of a compound according to claim 1.

**8.** A method of treating pain, comprising administering an effective amount of a compound according to claim 1.

**9.** A method of treating inflammation, comprising administering an effective amount of a compound according to claim 1.

# INTERNATIONAL SEARCH REPORT

International Application No PCT/JP92/00262

**I. CLASSIFICATION OF SUBJECT MATTER** (if several classification symbols apply, indicate all) [6]

According to International Patent Classification (IPC) or to both National Classification and IPC

Int. $Cl^5$ C07C43/23

**II. FIELDS SEARCHED**

Minimum Documentation Searched [7]

| Classification System | Classification Symbols |
|---|---|
| IPC | C07C43/23 |

Documentation Searched other than Minimum Documentation to the Extent that such Documents are Included in the Fields Searched [8]

Jitsuyo Shinan Koho 1926 - 1992
Kokai Jitsuyo Shinan Koho 1971 - 1992

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 1-207256 (Tsumura Corp.), August 21, 1989 (21. 08. 89), Columns 1 and 2 & WO, A, 89/03376 & EP, A, 334967 | 1-9 |
| A | Journal of Chromatography, Vol. 360, (1986), C. CHEN, et al. "Chromatographic analyses of isomeric shogaol compounds derived from isolated Gingerol Compounds of Ginger (Zingiber Officinale Roscoe)" P. 175-184 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 20, 1992 (20. 04. 92) | May 19, 1992 (19. 05. 92) |
| **International Searching Authority** | **Signature of Authorized Officer** |
| Japanese Patent Office | |